# EUROPÄISCHE PATENTANMELDUNG

(11) **EP 1 273 954 A2**
(43) Veröffentlichungstag der Anmeldung: **08.01.2003**
(21) Anmeldenummer: 02102004.5
(22) Anmeldetag: 05.07.2002
(51) Int. Cl.: G02B 21/24

(54) **Stativ**

(30) Priorität: 06.07.2001 DE 10133018
(71) Anmelder: Leica Microsystems (Schweiz) AG, 9435 Heerbrugg (CH)
(72) Erfinder: Sander, Ulrich, 9445, Rebstein (CH)
(74) Vertreter: Reichert, Werner F., Dr.

(57) **Zusammenfassung**

Die Erfindung betrifft ein Stativ, insbesondere für Operationsmikroskope, bei welchem motorisch angesteuerte Balancierverstellungen (6, 8, 12) für eine zusätzliche Positionierbarkeit in den drei Raumachsen X, Y und Z vorgesehen sind und dabei eine kompakte und kostensparende Bauweise erlauben.

## Beschreibung

Manche bekannten Stative - z.B. solche für Operationsmikroskope für die Ophthalmologie - tragen an ihrem freien Ende zwischen dem Mikroskop und dem vertikalen Stativträger eine X-Y-Verschiebeeinheit für das Mikroskop. Diese Verschiebeeinheit dient dazu, das Mikroskop im Millimeterbereich in X-Y-Richtung genau zu positionieren. Eine solche Anordnung der X-Y-Verschiebeeinheit ist in der Regel für einen Operateur störend, weil dabei eine große Masse und ein großes Volumen bewegt werden müssen. Außerdem ist es schwierig, damit die nötigen Sterilitätserfordernisse dauerhaft zu erfüllen. Zudem erhöht die X-Y-Verschiebeeinheit das Gewicht am Auslegerarm deutlich und muss in der Regel über ein entsprechendes Ausgleichsgewicht kompensiert oder durch einen entsprechend großen Stativfuß abgestützt werden. In der Folge müssen damit auch die gesamte Tragarmkonstruktion des Stativträgers und gegebenenfalls auch die gesamte Stativfußkonstruktion stärker bemessen bzw. großflächiger dimensioniert sein.

Einer Patentanmeldung der Anmelderin (WO-A-00/08508) lag somit die Aufgabe zugrunde, einen Stativaufbau zu finden, bei dem die X-Y-Verstellfunktion erhalten bleibt, jedoch eine deutliche Gewichtserhöhung der Tragarmkonstruktion und die sich daraus ergebenden weiteren Nachteile vermieden werden.

Die Lösung dieser alten Aufgabe sollte dabei nicht zwingend auf eine lineare Verstellung in zwei hintereinander ablaufenden Stufen oder generell auf eine lineare Verschiebung eingeschränkt sein. Durch sie waren auch beliebige, z.B. berechnete oder gesteuerte Kurven-, Dreh- bzw. Schwenkbewegungen in einer Horizontalebene und gegebenenfalls auch Z-Verstellungen in der Vertikalen mit umfasst.

Die Lösung der alten Aufgabe lag in der erfindungsgemäßen Verlagerung der X-Y-Verschiebeeinheit zumindest näher zum vertikalen Stativständer, sodass die X-Y-Positioniereinheit nicht nur das Mikroskop, sondern auch wenigstens einen Teil des horizontalen Stativträgers bewegt.

Bei der erwähnten WO-A-00/08508 war die X-Y-Verschiebeeinheit nicht zwingend auf schlittenförmige Verschiebebahnen eingeschränkt. Sie konnte z.B. auch aus wenigstens zwei motorisch angetriebenen Gelenken des horizontalen Stativträgers bestehen, deren aufeinander abgestimmte Schwenkbewegungen eine beliebige Lageveränderung des Mikroskops in der X-Y-Ebene erlauben.

Dieses waren Maßnahmen, die sich bei Operationsmikroskopen in der Ophthalmologie bewährt haben, wo es - wie schon eingangs erwähnt - auf eine möglichst präzise X-Y-Positionierung des Mikroskops während der Operation ankommt. Diese konnten in einer bevorzugten Ausgestaltung mit einer planaren motorischen X-Y-Verstellung realisiert werden, welche nicht direkt über dem Mikroskop platziert ist. Das Mikroskop selbst befand sich beim Aufbau gemäß der WO-A nicht auf einem Schwenkträger und wurde somit praktisch nur über die X-Y-Verstellung oder durch Schwenken des Stativ-Tragarms in seiner Lage verändert.

In der HNO-Heilkunde oder der Neurologie hingegen ist weniger eine möglichst genaue X-Y-Verstellung, sondern eher eine leichtgängige, möglichst einhändige Bedienung des Mikroskops - z.B. an einem Schwenkträger - gefordert. Insofern bieten Erkenntnisse aus der in der Ophthalmologie eingesetzten Mikroskop-Varianten keine Hilfestellung für Stative im HNO-Bereich.

Der Anmelderin ist es gelungen, speziell diesen Markt mit einem Operationsmikroskop mit der Bezeichnung "M400-E" zu bedienen, welches mit einer manuellen X-Y-Z-Verstellung und einem Schwenkträger ausgestattet ist. Diese ermöglichen es, das Mikroskop selbst oder mit den verschiedenen Adaptern und/oder Zusätzen in eine ausbalancierte Lage zu bringen, in welcher dann die geforderte leichtgängige Ein-Hand-Bedienung gewährleistet ist.

Von diesem Stand der Technik ausgehend, stellte sich die Frage, ob die Positionierverstellungen des Ophthalmo-Mikroskops in die Balancierverstellungen des HNO-Mikroskops verbessert und beschleunigt werden können und ob die (manuelle) aufwändige Ausbalancierung des Mikroskops vereinfacht und besser wirksam gemacht werden könne.

Die Lösung der oben angegebenen Aufgabe ergibt sich aus den Merkmalen des Hauptanspruchs. Durch die Erfindung gelingt es, das Gewicht und das Volumen am Lastarm des Stativs zu reduzieren und derart die Stativkonstruktion insgesamt zu verkleinern bzw. zu verbessern. Eventuelle Ausgleichseinrichtungen und gegebenenfalls auch der Stativfuß werden dimensionell verkleinert und leichter.

Die erfindungsgemäße Lösung dieser Aufgabe konnte somit in einer "Zweckentfremdung" der zur Balancierung gedachten manuellen X-Y-Verstellung gefunden werden, dahingehend, dass sie ihre alte Balancierfunktion beibehalten, aber zusätzlich eine neue, "artfremde" Aufgabe "aufgebürdet" bekommt. Diese neue Aufgabe ist das Positionieren des Mikroskopkörpers im Raum bzw. über dem zu untersuchenden Objekt. Erfindungsgemäß ist nun die manuelle Balancierverstellung als eine motorische Positionierverstellung ausgelegt. Hierfür wurde die eingebaute manuelle X-Y-Z-Schlittenverschiebung aus dem bekannten Mikroskop"M400-E" als motorisch angesteuerte planare X-Y-Verstellung und motorisch angetriebene Z-Verstellung (Fokussierung) ausgelegt.

Auf diese Art und Weise entstand ein Operationsmikroskop, welches sich in einem ausbalancierten Zustand leichtgängig und einhändig bewegen lässt und darüber hinaus gleichzeitig und motorisch exakt und schnell verstellt werden kann.

Teil der Erfindung ist es weiterhin, dass bei einer besonderen Ausgestaltung auch die Z-Achse in Form einer motorisch angetriebenen Fokussierung mit integriert ist.

Vorteilhaft im Sinne der erfindungsgemäßen Lösung sind die schon erwähnte Beschleunigung und Wirkungserhöhung, aber auch eine sehr kompakte Bauweise, weil eine separate X-Y-Kupplung obsolet wird. Je nach Ausgestaltung und Platzierung der X-Y-Verstellung verbleibt nämlich unter Umständen nur noch die motorische Fokussierung am Mikroskopkörper. Die übrigen Teile werden erfindungsgemäß in die Halterung (Träger) - jedenfalls aber in die Nähe des Mikroskopkörpers - integriert.

Verschiedene Ausgestaltungen und Anordnungen der X-, der Y- und der Z-Steuerungen sind denkbar. Nach einer bevorzugten Ausgestaltung der Erfindung ist die Verstellmöglichkeit entlang der Achsen Y und Z direkt am Mikroskopkörper angeordnet und die X-Verstellung befindet sich am Schwenkträger oberhalb des Mikroskops.

Ein nicht näher dargestellter Rechner dient gemäß einer weiteren Verbesserung in Verbindung mit geeigneten Sensorelementen dazu, die jeweilige Lage des Mikroskops in Bezug auf das Objekt bzw. auf einen Patienten zu erfassen und die X-Y-Z-Befehlswerte jeweils so umzurechnen, dass die ausgeführte Verstellbewegung z.B. im X-Y-Z-Koordinatensystem des Objekts bzw. Patienten oder im Koordinatensystem des Mikroskops durchgeführt wird; unabhängig davon, wie die Schwenklage des Mikroskops im Raum ist oder um das Balanceverhalten zu ermitteln und ggf. zu korrigieren.

Gemäß einer weiteren Ausgestaltung der Erfindung können die Verstelleinrichtungen auch in ein Deckenstativ eingebaut werden. Der Schutzbereich wäre demnach auch darauf erstreckt, wobei dann unter dem Begriff Ständer oder Stativ Hängeträger zu verstehen wären und unter Fuß eine beliebige Basis am Boden, der Wand oder der Decke.

Darüber hinaus sind gemäß einer weiteren Ausgestaltung die Verstelleinheiten abkoppelbar ausgeführt, so dass ein manueller Betrieb immer gewährleistet ist.

Eine weitere bevorzugte Ausgestaltung sieht vor, dass Elektromotoren mit integrierten Inkrementalgebern zum Einsatz gelangen. Dies ermöglicht es, dass Bezugskoordinaten einer bestimmten gewünschten oder ausbalancierten Lage automatisch angefahren werden.

Weitere Merkmale der Erfindung und Ausgestaltungsvarianten sind in den Unteransprüchen beschrieben und in den Figuren dargestellt. Hierbei bedeuten gleiche Bezugszeichen gleiche Bauteile; Bezugszeichen mit unterschiedlichen Indizes bedeuten Bauteile mit gleichen Aufgaben, jedoch unterschiedlichen konstruktiven Ausgestaltungen.

Es zeigen dabei schematisch:
- Fig. 1:: den Gesamtaufbau eines Operationsmikroskops mit Stativfuß, Vertikalträger, Steuereinheit, Horizontalträger, Schwenkträger und Mikroskopeinheit;
- Fig. 2:: in perspektivischer Darstellung einen Aufbau der Verstelleinheiten, des Schwenkträgers, der Mikroskopeinheit und eines Griffes zur bequemen Ein-Hand-Bewegung und
- Fig. 3:: einen Aufbau gem. Fig. 2, mit dem Unterschied, dass hier die X-Verstellung in der Achse A angeordnet ist.

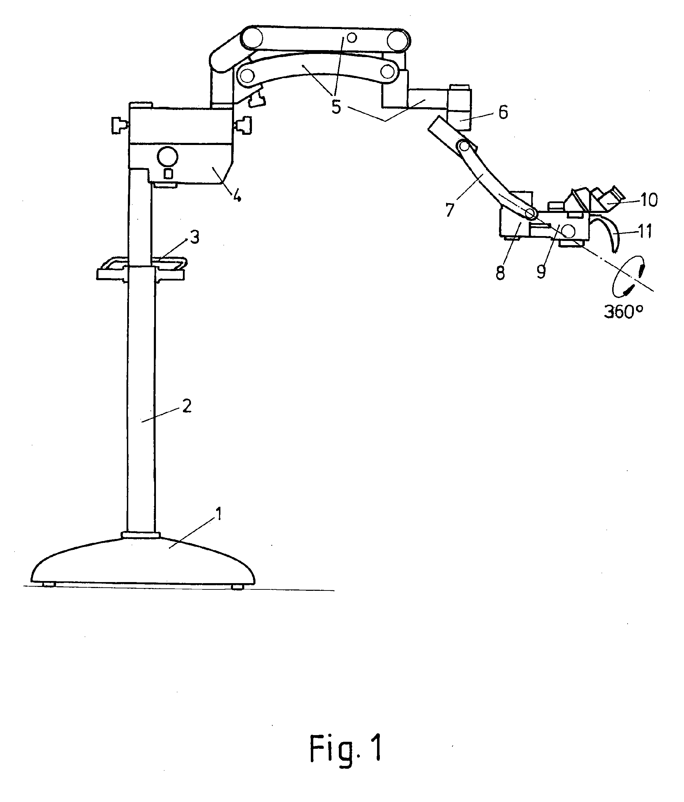

In Fig. 1 ist ein Gesamtaufbau eines erfindungsgemäßen Operationsmikroskops dargestellt. Hierbei ist ein Stativfuß 1 zu sehen, aus dem ein Vertikalträger 2 herausragt. Zur bequemen Fortbewegung des auf Rollen gelagerten Stativs ist am Vertikalträger 2 ein Griff 3 angebracht. Auf dem Vertikalträger 2 ist eine Steuereinheit 4 montiert, auf welcher sich die Horizontalträger 5 abstützen. In der dargestellten Ausführung enden die Horizontalträger in der X-Verstelleinheit 6, welche inklusive des Schwenkträgers 7 den daran befestigten Mikroskopkörper 9 in der X-Achse bewegt. Am anderen Ende des Schwenkträgers 7 befindet sich die Y-Verstellung 8. Des Weiteren sind noch der Binokulartubus mit den Okularen 10 und ein ergonomisch geformter Handgriff 11 dargestellt.

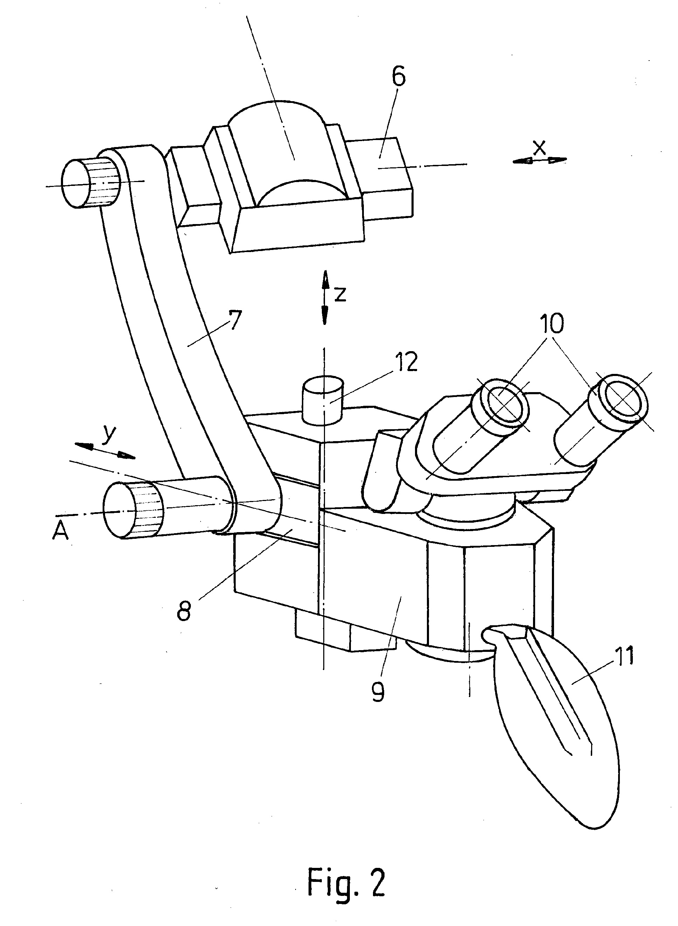

In Fig. 2 ist der Aufbau des Operationsmikroskops rund um den eigentlichen Mikroskopkörper 9 perspektivisch dargestellt. Hierbei ist die X-Verstelleinheit 6 am oberen Ende des Schwenkträgers 7 dargestellt und die Verstelleinheit 8 für die Y-Achse befindet sich am unteren Ende. Gleichzeitig ist hier auch eine Z-Verstellung 12 dargestellt. Vom Mikroskop selbst sind erneut der Körper 9, der Binokulartubus mit den Okulartuben 10 und der ergonomisch geformte Handgriff 11 zu sehen.

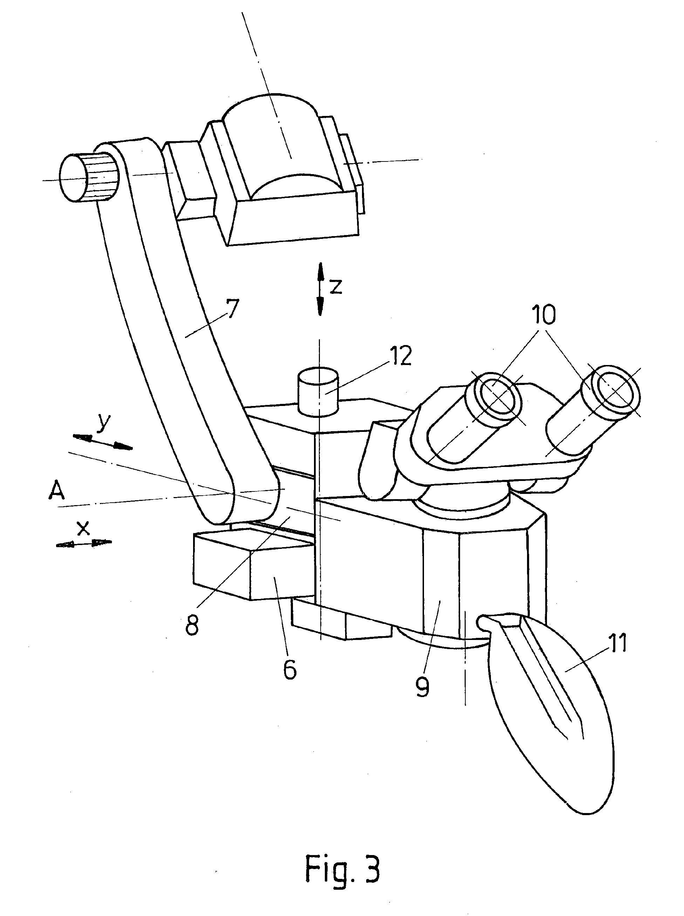

Fig. 3 zeigt einen anderen Anbringungsort für die X-Verstellung, nämlich auch wie die Y- und die Z-Verstellung direkt am Mikroskopkörper entlang der Achse A.

Beim Betrieb des Operationsmikroskops gewährleisten somit die drei zur Balancierung gedachten Verstellungen, dass das Mikroskop positioniert werden kann und Bewegungen in den drei Hauptachsen vollzogen werden können. Unter den drei Hauptachsen sind unter der X-Achse in etwa eine dem Betrachter nach links und rechts, unter der Y-Achse eine nach vorn und hinten und unter der Z-Achse eine nach oben und unten gerichtete Bewegungsrichtung zu verstehen. Natürlich kann dabei aus einem kombinierten Betrieb der Verstellungen, gekoppelt mit einer 360°-Drehbarkeit des Mikroskopkörpers jede erdenkliche Bewegung im Raum mittels des Ein-Hand-Griffes ausgeführt werden.

### Bezugszeichenliste

- 1:: Stativfuß
- 2:: Vertikalträger
- 3:: Griff
- 4:: Steuereinheit
- 5:: Horizontalträger
- 6:: X-Verstelleinheit
- 7:: Schwenkträger
- 8:: Y-Verstelleinheit
- 9:: Mikroskopkörper
- 10:: Okular(e)
- 11:: Handgriff
- 12:: Z-Verstelleinheit
- A:: Achse
- X:: X-Achse
- Y:: Y-Achse
- Z:: Z-Achse

## Patentansprüche

1. Stativ, insbesondere für Operationsmikroskope, mit einem Stativfuß (1), einem Vertikalständer (2), einem oder mehreren Horizontalträgern (5) und einem Schwenkträger (7), sowie einer X-Y-Z-Verstelleinheit zur Balancierung in einem Schwenkträger mit motorisch angesteuerten X-, Y- und Z-Verstelleinheiten (6, 8 und 12), **dadurch gekennzeichnet , dass** die X-Y-Z-Verstelleinheiten (6, 8 und 12) so ansteuerbar sind, dass sie den Mikroskopkörper (9) im Raum positionieren.

2. Stativ nach Anspruch 1, **dadurch gekennzeichnet, dass** die motorische Z-Verstelleinheit (12) als Fokussierung benutzt wird.

3. Stativ nach einem der vorhergehenden Ansprüche, **dadurch gekennzeichnet, dass** die motorische X-Verstelleinheit (6) oberhalb des Schwenkträgers (7) angeordnet ist.

4. Stativ nach einem der vorhergehenden Ansprüche, **dadurch gekennzeichnet, dass** die X-Verstelleinheit (6) am Mikroskopkörper (9) angeordnet ist.

5. Stativ nach einem der vorhergehenden Ansprüche, **dadurch gekennzeichnet, dass** die X-Y-Verstellung von (Balancier-) Stellmotoren (6 und 8) ausführbar ist und eine separate X-Y-Kupplung entfällt.

6. Stativ nach einem der vorhergehenden Ansprüche, **dadurch gekennzeichnet, dass** ein Rechner zur Koordinierung der X-Y-Z-Verstellungen (6, 8, 12) vorgesehen ist, welcher im Betrieb die Schwenkbewegungen des Mikroskopkörpers (9) um seine eigene vertikale oder um eine vertikale Achse des Stativs erfasst und bei einer X-Y-Verstellung berücksichtigt, so dass für den Anwender die X-Y-Richtung in Bezug auf ein Objekt bzw. auf einen Patienten immer gleich sind.

7. Stativ nach einem der vorhergehenden Ansprüche, **dadurch gekennzeichnet, dass** die Verstelleinheiten (6, 8, 12) abkoppelbar sind und dass ein manueller Notbetrieb vorgesehen ist.

8. Stativ nach einem der vorhergehenden Ansprüche, **dadurch gekennzeichnet, dass** anstelle des Stativfußes (1) eine Deckenoder Wandhalterung vorgesehen ist.

9. Verwendung einer X-Y-Z-Balancierverstelleinheit für das X-Y-Z-Positionieren eines Mikroskopkörpers (9) über einem Objekt.
